# EUROPÄISCHE PATENTSCHRIFT

(11) **EP 0 590 457 B1**
(45) Veröffentlichungstag und Bekanntmachung des Hinweises auf die Patenterteilung: **06.12.2000**
(21) Anmeldenummer: 93115086.6
(22) Anmeldetag: 20.09.1993
(51) Int. Cl.: C07D 277/78, C08K 5/47, C08L 21/00

(54) **Benzothiazolderivate als Vulkanisationsbeschleuniger, die sich zur Einführung von polaren Substituenten eignen**
Benzothiazole derivatives as vulcanisation accelerators suitable for the introduction of polar substituents
Dérivés de benzothiazole comme accélérateurs de vulcanisation appropriés pour l'introduction de substituants polaires

(30) Priorität: 02.10.1992 DE 4233197
(43) Veröffentlichungstag der Anmeldung: 06.04.1994
(73) Patentinhaber: BAYER AG, 51368 Leverkusen (DE)
(72) Erfinder: Scholl, Thomas, Dr., D-51469 Bergisch Gladbach (DE); Weidenhaupt, Hermann-Josef, Dr., D-52388 Nörvenich (DE); Kelbch, Stefan, Dr., D-51519 Odenthal-Erberich (DE); Engels, Hans-Wilhelm, Dr., D-50170 Kerpen (DE)

(56) Entgegenhaltungen:
- EP-A- 0 392 052
- FR-A- 2 430 943
- JOURNAL OF ORGANIC CHEMISTRY. Bd. 47, Nr. 5 , 26. Februar 1982 , EASTON US Seiten 765 - 767 MARIO D. BACHI ET AL 'Regiospecific thermolysis ot two diastereomeric beta-lactam sulfoxides'
- : "", RUBBER CHEM. TECHNOL. , , , Band 46, Nr. 5, Seiten 1299 - 1315

## Beschreibung

Die vorliegende Erfindung betrifft neue, zur Einführung von polaren Substituenten geeignete Vulkanisationsbeschleuniger sowie die Verwendung der neuen Vulkanisationsbeschleuniger zur Herstellung von mit seitenständigen polaren Gruppen modifizierten Kautschukvulkanisaten.

Die Herstellung von mit seitenständigen polaren Gruppen (z.B. OH-Gruppen) modifizierten Kautschukvulkanisaten ist beispielsweise aus der DE-A-2 653 144 und der EP-A-464 478 bekannt Die gemäß den beiden genannten Patentveröffentlichungen durch Mercaptane, wie 2-Mercapto-ethanol und Thioglykolsäure, modifizierten Kautschukvulkanisate weisen, insbesondere wenn sie mit Kieselsäure gefüllt sind, bessere mechanische Eigenschaften auf als die unmodifizierten mit Kieselsäure gefüllten Vulkanisate. Nachteilig bei den aus den vorgenannten Veröffentlichungen bekannten Verfahren zur Herstellung von modifizierten Kautschukvulkanisaten ist, daß die Herstellung der modifizierten Kautschuke und deren Vulkanisation in zwei getrennten Verfahrensschritten durchgeführt werden müssen, was zu Lasten der Wirtschaftlichkeit der Verfahren geht.

Weiterhin sind aus der DE-A-2 255 577 Organosilane einer speziellen Struktur bekannt, die als Additive für silikatische Füllstoffe enthaltende Kautschukmischungen dienen und die die Eigenschaften der Vulkanisate in überraschender und maßgebender Weise günstig beeinflussen sollen. Die in der DE-A-2 255 577 beschriebenen Additive besitzen keinerlei beschleunigende Wirkungen für die Vulkanisation, sondern erfordern zur Erzielung einer praxistauglichen Kinetik und Vernetzungsdichte zusätzliche Mengen eines Vulkanisationsbeschleunigers.

Schließlich wird in Rubber Chem. Technol. 46 (5), Seite 1299-1315 die Herstellung und das kinetische Verhalten von asymmetrischen Disulfiden in Kautschukvulkanisaten beschrieben. Die dort genannten Verbindungen, wie Cyclohexyl-dithiobenzthiazol, besitzen einen unpolaren und damit nicht zur Wechselwirkung mit Füllstoffen befähigten Cyclohexyl-Rest, und sind wie eigene Versuche belegen, wegen zu geringer Reaktivität und erheblichen Geruchsbelästigungen bei der Vulkanisation für selbige ungeeignet.

Gegenstand der vorliegenden Erfindung sind also neue Vulkanisationsbeschleuniger der Formel 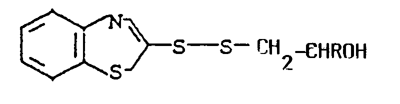 worin steht,
RH, C₁-C₁₂-Alkyl oder Cyclohexyl bedeutet.

Als C₁-C₁₂-Alkylreste in der obigen Formel kommen bevorzugt der Methyl-, Ethyl-, Propyl-, Butyl-, Octyl- sowie Dodecylrest in Frage.

Als bevorzugte neue Vulkanisationsbeschleuniger sind solche der nachstehenden Formeln zu nennen:

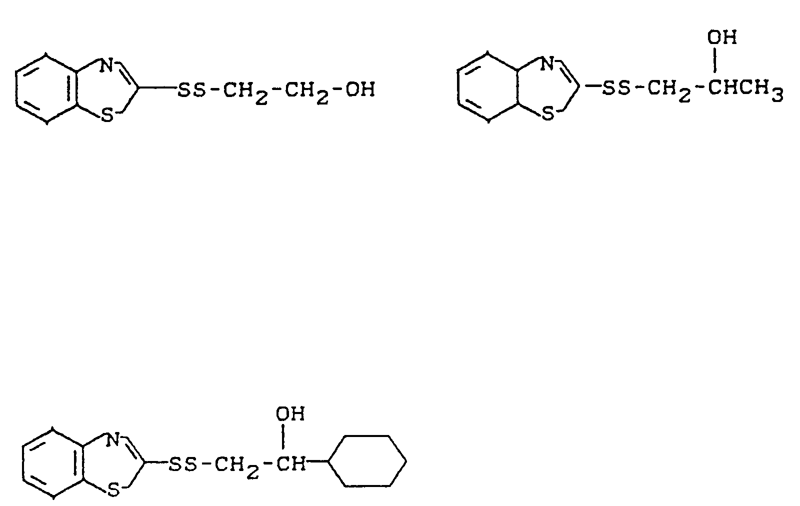

Die neuen Vulkanisationsbeschleuniger werden in Mengen von 0,1 bis 10 Gew.-%, bevorzugt 0,3 bis 4 Gew.-%, bezogen auf den Kautschuk der Kautschukmischung vor der Vulkanisation, eingesetzt.

Die erfindungsgemäßen Vulkanisationsbeschleuniger sind, wie bereits erwähnt, in der Lage während der Vulkanisation die Seitenkette

-S-CH₂CHROH

auf das Kautschukpolymerisat zu übertragen und so eine Modifizierung der Polymerkette mit polaren Substituenten herbeizuführen.

Die Herstellung der neuen Vulkanisationsbeschleuniger kann prinzipiell auf drei Wegen durchgeführt werden:
1. Umsetzung von Mercaptobenzthiazol-sulfenchloriden mit entsprechend substituierten Mercaptanen gemäß Reaktionsschema A: 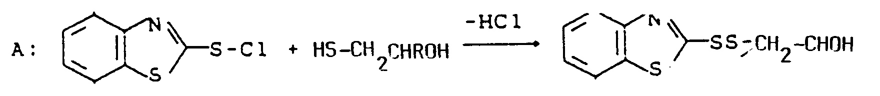
   Die Reaktion wird bevorzugt durchgeführt im Temperaturbereich von -20 bis +50°C ggf. in Gegenwart von Basen zum Abfangen des freiwerdenden Chlorwasserstoffs. Derartige Reaktionen sind z.B. beschrieben in Houben-Weyl, Methoden der Organischen Chemie, Thieme Verlag Stuttgart, Band E 11, Seite 140-142 (1985).
2. Aus Thioimiden, durch Umsetzung mit entsprechend substituierten Mercaptanen gemäß Reaktionsschema 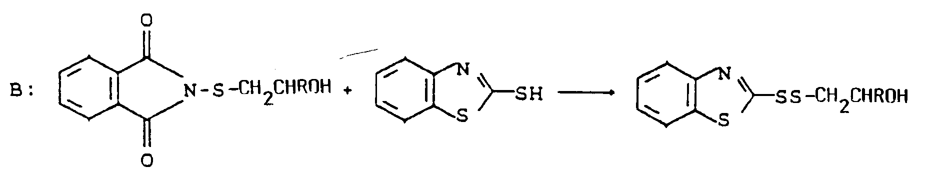 B:
   Die Reaktionen werden bevorzugt bei Temperaturen von 60 bis 120°C und in aromatischen Lösungsmitteln durchgeführt. Derartige Umsetzungen sind z.B. beschrieben in Tetrahedron Letters 41 (1970), 3551-3554.
3. Durch Umsetzung von 2,2'-Dithiobenzthiazol mit Mercaptanen, vorzugsweise in Gegenwart katalytisch wirksamer Basen und in aprotischen Lösungsmitteln bei Temperaturen von 50 bis 130°C gemäß Reaktionsschema C: 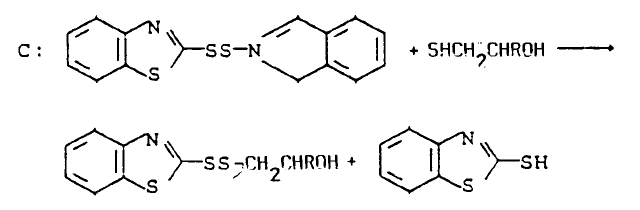
   Derartige Umsetzungen sind z.B. beschrieben in Houben-Weyl, Methoden der Organischen Chemie, Thieme Verlag Stuttgart, Band E11, Seite 146 (1985).

Die neuen erfindungsgemäßen Vulkanisationsbeschleuniger eignen sich für die Vulkanisation sowohl von Naturkautschuk als auch von Synthesekautschuken. Als Synthesekautschuke seien beispielsweise solche genannt wie sie bei W. Hoffmann, Kautschuk-Technologie, Gentner-Verlag, Stuttgart 1980, beschrieben sind. Sie umfassen u.a. Polybutadien (BR), Butadien/Acrylsäure-C₁₋₄-alkylester-Copolymerisate (ABR), Polychloropren(CR), Polyisopren(IR), Styrol/Butadien-Copolymerisate mit Styrolgehalten von 1 bis 60, vorzugsweise 20 bis 50 Gew.-% (SBR), Isobutylen/Isopren-Copolymerisate(IIR), Butadien/Acrylnitril-Copolymerisate mit Acrylnitrilgehalten von 5 bis 60, vorzugsweise 10 bis 50 Gew.-% (NBR), teilhydrierter oder vollständig hydrierte NBR-Kautschuke (HBNR), Ethylen/Propylen/Dien-Copolymerisate (EPDM) sowie Mischungen dieser Kautschuke.

Die neuen Vulkanisationsbeschleuniger können bei der Vulkanisation mit weiteren Kautschukhilfsprodukten eingesetzt werden, wie zusätzliche Beschleuniger, Alterungsschutzmittel, Wärmestabilisatoren, Lichtschutzmittel, Ozonschutzmittel, Verarbeitungshilfsmittel, Weichmacher, Tackifier, Treibmittel, Farbstoffe, Pigmente, Wachse, Streckmittel, organische Säuren, Verzögerer, Metalloxide und Aktivatoren, wie Triethanolamin, Polyethylenglykol und Hexantriol, die dem Fachmann der Gummiindustrie bekannt sind, Die Kautschukhilfsprodukte werden in den üblichen Mengen bei der Vulkanisation den Kautschuken zugesetzt.

Wie bereits erwähnt, eignen sich die neuen Vulkanisationsbeschleuniger insbesondere für die Herstellung von Kautschukvulkanisaten, die mit silikatischen Füllstoffen gefüllt sind Als silikatische Füllstoffe kommen beispielsweise in Frage: hochdisperse Kieselsäuren, hergestellt z.B. durch Fällung von Lösungen von Silikaten oder Flammenhydrolyse von Siliciumhalogeniden mit spezifischen Oberflächen von 5 bis 1000, vorzugsweise 20 bis 400 m²/g (BET-Oberfläche) und Primärteilchengrößen von 100 bis 400 nm. Die Kieselsäuren können gegebenenfalls auch als Mischoxide mit anderen Metalloxiden wie Aluminium-, Magnesium-, Calcium-, Barum-, Zink-, Zirkonium-, Titan-Oxide vorliegen. Geeignet sind weiterhin synthetische Silikate, wie Aluminiumsilikat, Erdalkalisilikat, wie Magnesium- oder Calciumsilikat, mit BET-Oberflächen von 20 bis 400 m²/g und Primärteilchendurchmessern von 10 bis 400 nm. Außerdem eignen sich als Füllstoffe natürliche Silikate, wie Kaolin und andere natürlich vorkommende Kieselsäuren, sowie Glasfasern und Glasfaserprodukte (Matten, Stränge) oder Mikroglaskugeln.

Neben den erwähnten silikatischen Füllstoffen können auch die bekannten Russe verwendet werden. Solche Russe werden beispielsweise nach dem Flammruß, Furnace- oder Gasruß-Verfahren hergestellt und besitzen BET-Oberflächen von 20 bis 200 m²/g, wie SAF, ISAF, IISAF, HAF, FEF oder GPF-Russe.

Besonders günstig lassen sich die neuen Vulkanisationsbeschleuniger verwenden, wenn neben den silikatischen Füllstoffen, wie Kieselsäuren, Ruß bei der Vulkanisation von Kautschuken verwendet wird. Bei einer solchen Vulkanisation kann das Verhältnis von Kieselsäure zu Ruß in beliebigen Grenzen variiert werden. Aus reifentechnischer Sicht werden beispielsweise Kieselsäure/Ruß-Verhältnisse von 1:10 bis 1:2 (in Gewichtsteilen) verwendet.

Die erfindungsgemäßen Vulkanisationsbeschleuniger lassen sich auf den in der Kautschukindustrie üblichen Maschinen, wie Mischwalzen, Knetern und Kalandern, verarbeiten.

Die mit den erfindungsgemäßen Vulkanisationsbeschleuniger hergestellten Vulkanisate eignen sich beispielsweise zur Herstellung von Kfz-Reifen, Dichtungen, Treibriemen und Federbälgen.

### Beispiele

### A: Herstellung der erfindungsgemäßen Vulkanisationsbeschleuniger

### Beispiel 1

### 2-Hydroxyethyl-dithio-benzthiazol

In eine Suspension von 83 g (0,25 mol) 2,2'-Dithiobisbenzthiazol in 600 ml Chlorbenzol leitete man bei 0 bis 5°C 17,5 g (0,25 mol) Chlorgas ein. Dann tropfte man diese Lösung zu 39 g (0,5 mol) Mercaptoethanol und ließ 7 Stunden bei Raumtemperatur nachrühren Das ausgefallene Produkt wurde abfiltriert, mit 500 ml Methylenchlorid versetzt und mit 500 ml 5%iger NaHCO₃-Lösung gewaschen. Die organische Phase wurde eingedampft. Es resultieren 102 g eines gelbbraunen Öls, das nach kurzer Zeit kristallisierte. Fp.: 65 bis 68°C.

¹H-NMR (CDCl₃): 3.1 ppm: 2 Alkyl-Protonen (Triplett), 3,9 ppm: 2 Alkyl-Protonen (Triplett), 4,4 ppm: 1 Hydroxyl-Proton (Singulett), 7,3 bis 7,9 ppm: 4 aromatische Protonen (Multiplett).

| Elementaranalyse: | | C | H | N | S |
|---|---|---|---|---|---|
| | ber. | 44,4 | 3,7 | 5,8 | 39,5 % |
| | gef. | 44,4 | 3,7 | 5,6 | 39,1 % |

### Beispiel 2

### 2-Hydroxylpropyl-dithio-benzthiazol

In einer Suspension von 83 g (0,25 mol) 2,2'-Dithiobisbenzthiazol in 600 ml Chlorbenzol leitete man bei 0 bis 5°C 17,5 g (0,25 mol) Chlorgas ein Dann tropfte man diese Lösung zu 46 g (0,5 mol) 1-Mercapto-2-propanol und ließ 7 Stunden bei Raumtemperatur nachrühren. Das ausgefallene Produkt wurde abfiltriert und mit 500 ml Methylenchlorid und 500 ml 5%iger NaHCO₃-Lösung versetzt. Nach zweimaligem Waschen mit Wasser wurde die organische Phase eingedampft. Es resultieren 94 g eines gelbbraunen Öls.

¹H-NMR (CDCl₃): 1,3 ppm: 3 Methyl-Protonen (Dublett), 2,75 bis 3,2 ppm: 2 Methylen-Protonen (Multiplett), 4,0 bis 4,1 ppm: 1 Methin-Proton (Multiplett), 4,5 ppm: 1 Hydroxyl-Proton (Singulett), 7,2 bis 8,0 ppm: 4 aromatische Protonen (Multiplett).

### B: Prüfung auf Eignung als Vulkanisationsbeschleuniger

### Beispiel 3

### Kautschukmischung:

| | |
|---|---|
| Naturkautschuk TSR 5 (Malaysian Rubber) | 100 Gew.-Tle. |
| Ruß N 115 (Degussa) | 48 Gew.-Tle. |
| Stearinsäure | 2 Gew.-Tle. |
| Zinkoxid | 3,5 Gew.-Tle. |
| Ozonschutzwachs Antilux 110 (Bayer) | 1 Gew.-Tl. |
| oligomeres Trimethyldihydrochinolin (Vulkanox HS, Bayer) | 1 Gew.-Tl. |
| N-Isopropyl-N'-phenyl-p-phenylendiamin (Vulkanox 4010 NA, Bayer) | 1,5 Gew.-Tle. |
| Schwefel | s. Tabelle |
| Beschleuniger | s. Tabelle |

In einem Kneter wurden bei 140°C Innentemperatur und einer Drehzahl von 50 Upm Kautschuk-Mischungen der nachfolgenden Zusammensetzung hergestellt. Die Mischzeit betrug 5 Minuten. Die Zugabe von Schwefel und Beschleuniger erfolgte zum Schluß bei 50°C auf der Walze Die Vernetzung und Vernetzungskinetik erfolgte anschließend bei 150°C und wurde mit einem Vulkameter gem. DIN 53 529 innerhalb von 45 Minuten registriert. Gemessen wurde die Zeit bis zur einsetzenden Vulkanisation (t-s in Minuten), die Zeit bis zum Erreichen von 90 % der maximalen Vernetzung (t-90) und das maximale Drehmoment (F max in N) zum Zeitpunkt der höchsten Vernetzungsdichte.

Neben den erfindungsgemäßen Beschleunigern wurden folgende Vergleichsverbindungen mit geprüft:
Vergleich 1: 2,2'-Dithio-bisbenzthiazol (Vulkacit DM, Bayer)
Vergleich 2: Bis-triethoxysilylpropyl-tetrasulfid (DE 2.255.577)

**Tabelle 1:**

| Beschleuniger | Schwefel | t-s | t-90 | Fmax |
|---|---|---|---|---|
| 1.4 phr Bsp.1 | 1,8 phr | 3,6 | 12,9 | 42,3 |
| 1.4 phr Bsp.2 | 1,8 phr | 4,5 | 13,4 | 42,1 |
| 1.4 phr Vgl.1 | 1,8 phr | 2,5 | 9,2 | 39,7 |
| 1.4 phr Vgl.2 | 1,8 phr | Keine Vulkanisation | | |

Aus den Ergebnissen wird deutlich, daß die erfindungsgemäßen Verbindungen im Gegensatz zu der Vergleichsverbindung 2 eine beschleunigende Wirkung besitzen, so daß auf zusätzliche Beschleuniger verzichtet werden kann. Gegenüber dem bekannten Beschleuniger 2,2'-Dithio-benzthiazol (Vgl.-Beispiel 1) besitzen sie den Vorteil einer deutlich höheren Verarbeitungssicherheit (gemessen als Scorch-zeit t-s).

### C: Vulkanisate mit verbesserter Ermüdungsbeständigkeit und verbessertem dynamischem Dämpfungsverhalten

### Beispiel 4

Gemäß dem Verfahren und der Zusammensetzung aus Beispiel 8 wurde eine Reifenlaufflächen-Mischung hergestellt und bei 150°C/20 Minuten vulkanisiert. Die verwendeten Vernetzungssysteme wurde so eingestellt, daß die Vulkanisate die gleiche Vernetzungsdichte (gemessen als Modul bei 100 % bzw. 300 % Dehnung) aufweisen:
- A:: 1,8 phr Schwefel, 1,4 phr Beispiel 1
- B:: 1,8 phr Schwefel, 1,4 phr Beispiel 2
- Vergleich:: 1,2 phr Schwefel, 1,4 phr Morpholinomercaptobenzthiazolsulfenamid (Vulkacit MOZ, Bayer)

Die Ermüdungsbeständigkeit wurde durch Monanto-Fatigue-to-Failure bei 70°C bestimmt. Gemessen wurde die Anzahl Dehnungen bis zum Bruch der Prüfkörper.

**Tabelle 2:**

| | A | B | Vergleich |
|---|---|---|---|
| Festigkeit (MPa) | 29 | 29 | 30 |
| Bruchdehnung (%) | 510 | 520 | 515 |
| Modul 100 (MPa) | 2,3 | 2,2 | 2,2 |
| Modul 300 (MPa) | 13,0 | 11,9 | 12,0 |
| Ermüdungsbeständigkeit (in 100* Zyklen) | 475 | 770 | 265 |
| tan delta (0°C) | 0,209 | -- | 0,195 |
| tan delta (100°C) | 0,086 | -- | 0,108 |

Es wird deutlich, daß die dynamische Ermüdungsbeständigkeit der Kautschuk-Vulkanisate mit den neuen Beschleunigern erheblich verbessert wird Ferner zeigt das erfindungsgemäße Vulkanisat A gegenüber dem Vergleich eine höhere dynamische Dämpfung bei 0°C (gemessen als tan delta nach DIN 53513), die nach dem bekannten Wissenstand mit einer höheren Naßrutschfestigkeit einhergeht. Außerdem wird eine geringere dynamische Dämpfung bei 100°C erzielt, die bekanntermaßen einen geringeren Rollwiderstand bei Kfz-Reifen bewirkt.

### D: Füllstoff-Aktivierung von Kieselsäure-gefüllten SBR-Vulkanisaten

### Beispiel 5

In einem Kneter wurde bei 140°C Innentemperatur und einer Drehzahl von 50 Upm Kautschukmischungen der folgenden Zusammensetzung hergestellt. Die Mischzeit betrug 5 Minuten. Die Zugabe von Schwefel und Beschleuniger erfolgte zum Schluß bei 50°C. Anschließend wurden Prüfplatten der Stärke 1 mm durch 30-minütige Vulkanisation bei 160°C hergestellt.

### Mischung:

| | |
|---|---|
| SBR-Kautschuk Buna EM 1500 (HÜLS) | 70 Gew.-Tle. |
| SBR-Kautschuk Buna EM 1778 (HÜLS) | 41 Gew.-Tle. |
| Kieselsäure Vulkasil S (BAYER) | 50 Gew.-Tle. |
| Zinkoxid | 3 Gew.-Tle. |
| Stearinsäure | 2 Gew.-Tle. |
| Diethylenglykol | 1,5 Gew.-Tle. |
| Vulkanox OCD (BAYER) | 1 Gew.-Tl. |
| Cumaronharz | 5 Gew.-Tle. |
| Schwefel | 2 Gew.-Tle. |
| Beschleuniger | 1,5 Gew.-Tle. |

**Tabelle 3:**

| | Modul 300 (MPa) | Festigkeit (MPa) | Bruchdehnung (%) |
|---|---|---|---|
| A: Verbindung Bsp.1 | 3,8 | 17,5 | 1015 |
| B: Verbindung Bsp.2 | 3,0 | 14,5 | 1080 |

| Vergleich 1: | | | |
|---|---|---|---|
| Morpholino-mercapto-benzthiazol-sulfenamid | 1,9 | 10,5 | 1270 |

| Vergleich 2: | | | |
|---|---|---|---|
| 2-Benzthiazolyl-dithio-cyclohexan (Rubber Chem. Technol. 46 (5), 1299 bis 1315. Keine Vulkanisation. Starke Geruchsbelästigung. | | | |

Die Ergebnisse belegen, daß mit den erfindungsgemäßen Verbindungen eine höhere Vernetzungsdichte erzielt wird als mit dem Sulfenamid-Beschleuniger gemäß Vergleich 1. Dies wird auf eine verbesserte Wechselwirkung der mit Hilfe des Beschleunigers in die Kautschukkette eingeführten, polaren Gruppe mit dem Kieselsäure-Füllstoff zurückgeführt. Die nicht polar-substituierten Vergleichsverbindung (Vergleich 2) eignen sich für die Vulkanisation dieser Mischung überhaupt nicht. Es ließen sich keine Prüfkörper herstellen. Zudem trat eine starke Geruchsbelästigung durch freigesetzte Mercaptane auf.

## Patentansprüche

1. Zur Einführung von polaren Substituenten geeignete Vulkanisationsbeschleuniger der Formel 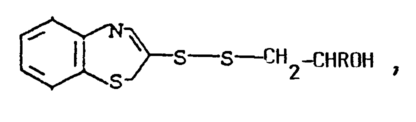 worin steht,
RH, C₁-C₁₂-Alkyl oder Cyclohexyl bedeutet.

2. Verwendung der Vulkanisationsbeschleuniger nach Anspruch 1 zur Herstellung von Kautschukvulkanisaten.

## Claims

1. Vulcanisation accelerators suitable for the introduction of polar substituents, corresponding to the formula 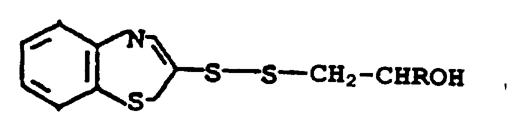 wherein R denotes H, a C₁-C₁₂ alkyl or cyclohexyl.

2. The use of the vulcanisation accelerators according to claim 1 for the production of vulcanised rubbers.

## Revendications

1. Accélérateurs de vulcanisation, appropriés à l'introduction de substituants polaires, de formule 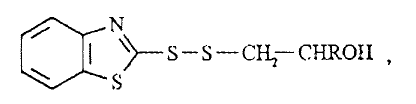 dans laquelle R représente H ou un reste alkyle en C₁-C₁₂ ou cyclohexyle.

2. Utilisation des accélérateurs de vulcanisation selon la revendication 1 pour la préparation de caoutchoucs vulcanisés.
